# EUROPEAN PATENT SPECIFICATION

(11) **EP 0 231 261 B1**
(45) Date of publication and mention of the grant of the patent: **06.04.1994**
(21) Application number: 86904583.1
(22) Date of filing: 24.06.1986
(51) Int. Cl.: A61K 9/00, A61K 9/42

(54) **MULTILAMELLAR LIPOSOMES HAVING IMPROVED TRAPPING EFFICIENCIES**
MULTILAMELLARE LIPOSOME MIT VERBESSERTER EINSCHLIESSUNGSWIRKUNG
LIPOSOMES MULTILAMELLAIRES PRESENTANT UNE EFFICACITE DE CAPTURE AMELIOREE

(30) Priority: 05.07.1985 US 752423; 21.11.1985 US 800545
(43) Date of publication of application: 12.08.1987
(73) Proprietor: THE LIPOSOME COMPANY, INC., Princeton, NJ 08540 (US)
(72) Inventor: BALLY, Marcel, B., Vancouver British Columbia V6T 1M8 (CA); CULLIS, Pieter, R., Vancouver British Columbia V6J 3R2 (CA); HOPE, Michael, J., Vancouver British Columbia V6R 2K2 (CA); JANOFF, Andrew, S., Yardley, PA 19067 (US); MAYER, Lawrence, D., Vancouver British Columbia V6N 2P3 (CA)
(74) Representative: Warcoin, Jacques
(86) International application number: US8601371
(87) International publication number: WO8700043

(56) References cited:
- EP-A- 0 002 835
- EP-A- 0 092 453
- EP-A- 0 171 710
- WO-A-85/00515
- WO-A-85/04326
- US-A- 4 016 100
- US-A- 4 397 846
- US-A- 4 515 736
- US-A- 4 532 089
- WO 86/01102
- WO 86/00238
- C.J. Kirby et al.: "Liposome technology", vol. I: "Preparation of liposomes", 1984, pp. 19-27, Chapter 2: "A simple procedure for preparing liposomes capable of high encapsulation efficiency under mild conditions" CRC Press Inc., Boca Raton, US
- The Merck Index, 10th Edition, 1983, p. 8686, Rahway, N.J. US
- Chemical Abstracts, vol. 103, no. 23, 9th December 1985, p. 27, abstract no. 189238t, Colombus, Ohio, US; J. Schacht et al.: "Aminoglycoside-cell receptor interactions: implications for toxicity and in vitro models"; & Proc. Int. Congr. Chemother. 13th, 1983, 5, 105/39-105/44
- Chemical Abstracts, vol. 105, no. 10, 8th September 1986, p. 405, abstract no. 85100s, Colombus, Ohio, US; B. Nagy et al. tudy on the sub-conjunctival application of liposome encapsulated tobramycin" & Ann. Immunol. Hung. 1985, 25, 355-63
- Biochimica et Biophysica Acta, 557, 1979, Elsevier/North Holland, Biomedical Press; Olson et al.: "Preparation of liposomes of defined size distribution by extrusion through polycarbonate membranes", see abstract

## Description

### TECHNICAL FIELD

The present invention is directed to liposomes. More particularly, the present invention is directed to multilamellar vesicles having improved trapping efficiency and equilibrium transbilayer solute distribution.

### BACKGROUND ART

Liposomes of the type which are multilamellar vesicles (MLV's) are usually formed by mechanical dispersion of dried lipid in an aqueous buffer. It is commonly assumed that this procedure results in an equilibrium interlamellar distribution of solutes present in the buffer. However, it has been demonstrated that the trapped buffer may have reduced solute concentrations resulting in osmotic imbalances between exterior and interior environments. Gruner et al., Biochemistry, 24, 2833-2842 (1985). These osmotic imbalances can lead to membrane potentials, transbilayer pH gradients and deformations due to osmotic forces. Equilibrium solute distributions can be achieved by techniques involving dispersion of the lipid in mixtures of organic solvent and aqueous buffer, where the organic solvent is subsequently removed under reduced pressure.

Alternative procedures have been described in U.S. Patent Nos. 4,522,803 and 4,588,578 to prepare liposomes having properties different than earlier MLV's.

The low trapping volume and trapping efficiency of MLV systems have presented difficulties in employing these systems in applications such as drug delivery. As a result, alternative procedures have been developed to prepare liposomal systems which involve the use of organic solvents or detergents as solubilizing agents. Organic solvents and detergents are undesirable ingredients in drug delivery systems.

It would, therefore, be desirable to obtain a multilamellar vesicle having high trapping efficiency, high trapped volumes and equilibrium transbilayer solute distribution prepared in the absence of organic solvents or detergents.

### DISCLOSURE OF INVENTION

We have prepared a new multilamellar vesicle dispersed in an aqueous phase comprising an aqueous medium, a trapping efficiency of at least about 30 percent, preferably about 40 percent and a lipid concentration of at least about 50 mg/ml. More preferably, the trapping efficiency is at least about 50 percent and preferably the lipid concentration is at least about 100 mg/ml, more preferably between about 100 and 1000 mg/ml, and still more preferably between about 100 and 400 mg/ml.

The vesicles of the present invention have an interlamellar equal solute distribution. The vesicle can contain a bioactive agent. The lipid can comprise phospholipid such as phosphatidylcholine, and may additionally comprise a sterol such as cholesterol. The vesicle can be concentrated by removal of all or part of the aqueous phase.

The multilamellar vesicles of the present invention can be prepared by dispersing (a) a lipid in an aqueous phase to form a multilamellar vesicle, (b) rapidly cooling the multilamellar vesicle to obtain a frozen lipid-aqueous medium mixture, and (c) warming the mixture. The rapid cooling step preferably employed liquid nitrogen. Steps (b) and (c) are repeated at least two times, preferably at least four times.

In addition, the resulting multilamellar vesicles of the present invention can be filtered to obtain a resulting vesicle dispersed in an aqueous phase comprising a lipid concentration of at least about 50 mg/ml of aqueous phase, a mean diameter of less than about 100 nanometers and a trapping efficiency of at least about 10%. Preferably the lipid concentration is at least about 100 mg/ml, more preferably about 100-400 mg/ml. The trapping efficiency is preferably about 40%. Preferably the resulting vesicles have a uniform size distribution.

Accordingly the present invention provides a process for preparing a multilamellar vesicle, containing a bioactive substance, having interlamellar equal solute distribution such as claimed in claims 1 to 6. The present invention also provides a multilamellar vesicle obtainable by this process such as claimed in claims 7 to 19.

### BRIEF DESCRIPTION OF THE DRAWINGS

FIG. 1 is an NMR spectrum of (A) an MLV in the absence of Mn²⁺, (B) an MLV prepared in the presence of 0.5 mM Mn²⁺, and (C) an MLV subject to five freeze-thaw cycles.

FIG. 2 is a graph showing the effect of the number of freeze-thaw cycles on the ³¹P NMR signal intensity.

FIG. 3 is shows freeze fracture electron micrographs of (A) MLV's and (B) FATMLV's of the present invention.

FIG. 4 is a graph showing the effect of the number of freeze-thaw cycles on trapped volume.

FIG. 5 is a graph showing the effect of cholesterol on the trapped volume of FATMLV's of the present invention.

FIG. 6 is a graph of percent ³¹P-NMR signal intensity remaining after Mn²⁺ addition for MLVs (A) and FATMLVs (B) passed through polycarbonate filters of defined pore sizes. MLV's and FATMLV's were prepared at 100 mg egg phosphatidyl choline/ml as described in Example 1. ³¹Phosporus-NMR signal intensities were determined before and after addition of Mn²⁺ (final concentration = 5mM) for vesicles passed the indicated number of times through 400 (closed circles), 200 (closed squares), 100 (open squares), 50 (open circles) and 30 (closed triangles) nm pore size filters.

FIG. 7 is a graph of aqueous trapped volumes of MLVs (closed circles) and FATMLVs (open circles) passed 20 times through polycarbonate filters of the indicated pore size. Vesicles were prepared at 100 mg lipid/ml of buffer containing ¹⁴C-inulin. The error bars represent the standard deviation determined from 3 samples.

FIG. 8 is a graph of percent trapping efficiency of FATMLVs passed through 400 (closed square), 100 (closed circle) and 50 (open circle) nm pore size filters as a function of phospholipid concentration. Vesicles were prepared in the presence of ²²Na as an aqueous marker. The values given represent the amount of ²²Na remaining associated with the vesicles after removal of free ²²Na relative to the total amount of ²²Na in the initial lipid dispersion.

### DETAILED DESCRIPTION

The following definitions will be employed:
liposome - a man-made structure composed of lipid bilayers that enclose a volume.
multilamellar vesicles (MLVs) - liposomes containing multiple lipid bilayers forming two or more shells.
FATMLV - a MLV which has been subject to at least one freeze-thaw cycle.
lipid - an agent exhibiting amphipathic characteristics causing it to spontaneously adopt an organized structure in water wherein the hydrophobic portion of the molecule is sequestered away from the aqueous phase.
freeze-thaw-cycle - cooling a liposome below the freezing point of the aqueous solvent contained within the liposome, then warming to a temperature whereby the aqueous medium or phase is melted.
trapping efficiency or encapsulation efficiency - the fraction of an aqueous phase sequestered by liposome bilayers when a lipid is dispersed in the aqueous phase; given as the percent of the original volume of the aqueous phase .
captured volume or trapped volume - the volume enclosed by a given amount of lipid with units of liters entrapped per mole of total lipid.
lipid concentration - the amount of lipid added per ml of aqueous phase; the units are generally mg/ml.

Multilamellar vesicles (MLV's) can be prepared by a number of methods. In one process, one or more selected lipids are deposited on the inside walls of a suitable vessel by dissolving the lipids in an organic solvent such as chloroform and then evaporating the organic solvent, adding an aqueous phase which is to be encapsulated to the vessel, allowing the aqueous phase to hydrate the lipid, and mechanically agitating, for example, swirling or vortexing, the resulting lipid suspension to produce the desired liposomes.

Alternatively, one or more selected lipids can be dispersed by employing mechanical agitation in an aqueous phase to produce MLV's. The process requires about 1-10 minutes at a temperature above the gel/liquid crystalline transition temperature.

The organic solvent can contain a bioactive agent such as a drug, preferably a bioactive agent which is both soluble in the organic solvent and is lipophilic.

The aqueous medium is that enclosed by lipid bilayers. Generally the aqueous medium will have the same constituents as the aqueous phase, although the amounts may be different. Although the following discussion is directed to an aqueous medium, it clearly also applies to the aqueous phase. The aqueous medium can be for example, water or water containing dissolved salt or buffer. The aqueous medium may contain a bioactive agent, preferably a bioactive agent which is water soluble. Water soluble bioactive agents which can be incorporated into FATMLV's of the present invention include antibacterial aminoglycosides such as tobramycin. Pilocarpine can be incorporated for ocular administration for treating glaucoma.

In the MLV the bioactive agent generally partitions between the aqueous and lipid portions of the liposome depending on the agent's lipophilic and hydrophilic character.

In addition, the MLV's produced by the procedures previously described have low trapped volumes and corresponding low trapping efficiencies, which causes the loss of valuable solutes in the aqueous solvent and the added cost of recycling the untrapped aqueous phase.

The lipids which can be employed in the present invention include cholesteryl hemisuccinate and salts thereof, tocopheryl hemisuccinate and salts thereof, a glycolipid, a phospholipid such as phosphatidylcholine (PC), phosphatidylethanolamine (PE), phosphatidylserine (PS), phosphatidylglycerol (PG), phosphatidic acid (PA), phosphatatidylinositol (PI) and sphingomylin (SPM), alone or in combination. The phospholipids can be synthetic or derived from natural sources such as egg or soy. Sterols such as cholesterol can be combined with the phospholipids. The phospholipids employed and the amount of sterol present depends on a number of factors such as lipophilicity of any added bioactive agent and the required properties of the liposome. These factors are well known to those skilled in the art.

The lipid concentration for the present invention is at least about 50 mg/ml. At lower concentrations, multilamellar vesicles of the present invention having a high trapping efficiency are difficult or impossible to form. A preferred lipid concentration is between about 100 and 1000 mg/ml, more preferably 100-600 mg/ml, and still more preferably 100-400 mg/ml.

A high trapping efficiency results in a large fraction of the aqueous phase being entrapped in the liposome. In the present invention, the liposomes have a trapping efficiency of at least about 30 percent, preferably about 40 percent, more preferably at least 50 percent and, most preferably about 50-90 percent. Generally, a lipid concentration of at least about 100 mg/ml is needed to obtain a trapping efficiency of at least about 50 percent.

The dispersed lipid mixture results in predominately MLV's. These MLV's have been shown to be in osmotic imbalance when a solute is present in the aqueous medium prior to addition of the lipid. For example, when Mn²⁺ ion from MnCl₂ is present in an aqueous buffer, non-equilibrium transmembrane distributions of the paramagnetic Mn²⁺ ion in the MLV's can be observed by NMR spectroscopy. Mn²⁺ is a "broadening" agent which quenches the ³¹P NMR signal arising from phospholipids with which the ion comes in proximity. When there are asymmetric transbilayer concentrations of Mn²⁺ in the MLV, the ³¹P NMR resomme arising from the phospholipids on either side of the bilayers are not equally quenched.

The freeze-thaw cycle of the present invention requires rapid freezing of the dispersed lipid medium mixture and then warming the frozen mixture in a constant temperature bath, to a temperature which will cause the aqueous phase to melt. The temperature employed is generally above the transition temperature for the gel-liquid crystalline transition. A constant temperature bath of about 25-50°C, preferably about 40°C, is generally effective.

Liquid nitrogen baths have been found to be particularly effective for the freezing step. A comparison was made of the FATMLV's prepared by placing the mixture in dry ice/ethanol and liquid nitrogen baths. For FATMLV's prepared by freezing liquid nitrogen, the trapping efficiency and the trapped volume was at least about 1.5-fold greater then when dry ice/ethanol was employed. At higher lipid concentrations, trapping efficiencies at least four-fold greater can be achieved. FATMLV's prepared using a dry ice/ethanol bath do not have the properties of those of the present invention. Strauss, "Freezing and Thawing of Liposome Suspensions," in Liposome Technology, Ed. G. Gregoriadis, Chapter 15, CRC Press, Inc., Boca Raton, Fla., 1984, discusses the rapid vs. slow cooling of liposome preparations.

The number of freeze-thaw cycles affects the properties of the resulting FATMLV. Generally, three or more freeze-thaw cycles are required to obtain an equilibrium interlamellar osmotic balance. The balance can be observed by following the effect of the number of freeze-thaw cycles upon the distribution of Mn²⁺ as measured by ³¹P NMR when the FATMLV includes phospholipid-containing bilayers.

When a lipid containing phospholipid solubilized in an aqueous phase by means of a detergent such as Triton X-100, the ³¹P NMR spectrum in the presence of Mn²⁺ shows all the phosphorous to be equally quenched. Essentially the same spectrum is observed for a FATMLV after three cycles.

About five freeze-thaw cycles in liquid nitrogen and a 40°C constant temperature bath, result in FATMLV's of the present invention. After five freeze-thaw cycles the morphology of the FATMLV changes. Before freeze-thawing, the MLV's exhibit the tightly packed "onion skin" arrangements of concentric bilayers normally associated with multilamellar liposomes. After five freeze-thaw cycles, a new structure is observed by freeze-fraction electron micrographs. The interlamellar spacing is increased by up to about five fold and closed lamellar systems can be intercalated between bilayers. The tightly packed arrangement is substantially absent.

The FATMLV's after at least about five cycles have significantly greater trapped volume and trapping efficiencies. The trapped volume per umol phospholipid can be determined using ²²Na⁺ as an aqueous marker. At 100 mg/ml phosphatidylcholine, more than an order of magnitude increase in trapped volume is observed after five freeze-thaw cycles compared to non-freeze-thawed MLV's. After about eight freeze-thaw cycles additional cycles do not result in any further significant changes in trapped volume.

The freeze-thaw protocol of the present invention is effective for various lipid compositions. For example, for a lipid whose components are egg PC containing between 0 and 50 mole percent cholesterol, substantially the same results with regard to trapped volume were obtained after five freeze-thaw cycles utilizing liquid nitrogen and 40°C water constant temperature bath. The actual number of freeze-thaw cycles needed will depend on the lipid composition and the method of preparation of the liposome employed as starting material for the freeze-thaw procedure of the present invention. The previously discussed procedures employing a marker can be readily employed to determine the number of freeze-thaw cycles required to obtain high entrapment which is substantially unchanged with additional freeze-thaw cycles.

The fraction of lipid in the outer bilayer in FATMLV's of the present invention is less than about 35 percent. When the lipid is egg PC at a lipid concentration of 50 mg/ml the fraction of lipid in the outer bilayer is about 32-34 percent. The fraction of lipid in the outer bilayer decreases with increasing lipid concentration, but is generally larger than that of MLV's or FATMLV's of the prior art. For egg PC (100 mg/ml) the outer bilayer fraction is about 26-30 percent, 200 mg/ml is about 13-17 percent, and 400 mg/ml is about 13-17 percent. Comparisons should be made for the same ingredients and formulations.

The aqueous phase of the dispersions of the present invention vesicle can be removed to form a concentrated FATMLV dispersion. Methods of removal include dialysis, centrifugation, dehydration and lyophilization. Dehydration is described in a copending U.S. patent application filed June 26, 1985, M. B. Bally et. al., "Encapsulation of Antineoplastic Agents in Liposomes," Serial No. 749,161 (WO 86/01102) and in a copending U.S. patent application filed July 26, 1985, A. S. Janoff et al., "Dehydrated Liposomes", Serial No. 759,419, (WO 86/01103).

Variable size vesicles can be prepared by a rapid extrusion technique where lipid dispersions are passed under moderate pressure, e.g., nitrogen at pressures up to about 800 psi 5.520 KPa through polycarbonate filters whose pore size can be varied from 30-800 nm or more. Methods relating to rapid extrusion techniques through polycarbonate filters and the use of the methods to obtain liposomes of uniform size distribution are described in the following copending United States patent applications: Cullis et al., Serial No. 622,690 (WO 86/002387), filed June 20, 1984; Cullis et al., Serial No. 622,502 WO 86/00238, filed June 20, 1984; and Cullis et al., "Extrusion Technique for Producing Unilamellar Vesicles", filed October 16, 1985, Serial No. 788,017(WO 86/00238).

Unilamellar vesicles with a large, e.g., (151 nm) mean diameter can be produced if FATMLV's of the present invention are passed through 200 nm pore size filters. This freeze-thaw process also increased the aqueous trapped volume of vesicles 1.5 to 3.0 times when filters with pore sizes ranging from 100 to 400 nm were utilized. Increasing the lipid concentration to 400 mg/ml of aqueous buffer for these systems increased the trapping efficiency levels to 80 and 50 percent for vesicle produced employing 400 and 100 nm pore size filters, respectively. Freeze fracture electron microscopy revealed that vesicles produced at the very high lipid concentrations exhibited negligible alterations in size distribution or extent of multilamellarity as compared to systems produced at lower lipid levels. These results suggest that the freeze-thaw extrusion protocol offers a very general and versatile method for producing vesicles of variable sizes and has several advantages that were unavailable with previous procedures. Sized vesicles containing drugs or other bioactive agents are useful for treating illnesses in mammals including humans. For example, sized vesicles containing pilocarpine may be used to treat glaucoma and those containing tobramycin may be used to treat infections in mammals, including humans.

A variety of unilamellar and multilamellar vesicles of differing sizes can be generated by extrusion of MLV's through polycarbonate filters with different pore sizes. It is convenient to introduce the general term "VET's" to indicate "vesicles by extrusion techniques" with a numerical subscript to indicate the pore size employed. Thus a VET₅₀ system indicates vesicles extruded through filters with 50 nm pore size, whereas a VET₄₀₀ system indicates extrusion through 400 nm pore size filters. Once the lamellarity and size of these systems has been determined, this nomenclature can be further refined to indicate the large or small character of unilamellar systems or the multilamellarity of other (e.g., large unilamellar vesicles by extrusion through 100 nm filters - LUVET₁₀₀'s; small unilamellar vesicles by extrusion through 50 nm filters - SUVET₅₀ 's; or multilamellar vesicles obtained by extrusion through 400 nm filters - MLVET₄₀₀'s). An arbitrary decision to designate unilamellar systems extruded through 100 nm or larger pores as "large" has been made, whereas systems extruded through 50 nm or smaller pores are indicated as "small".

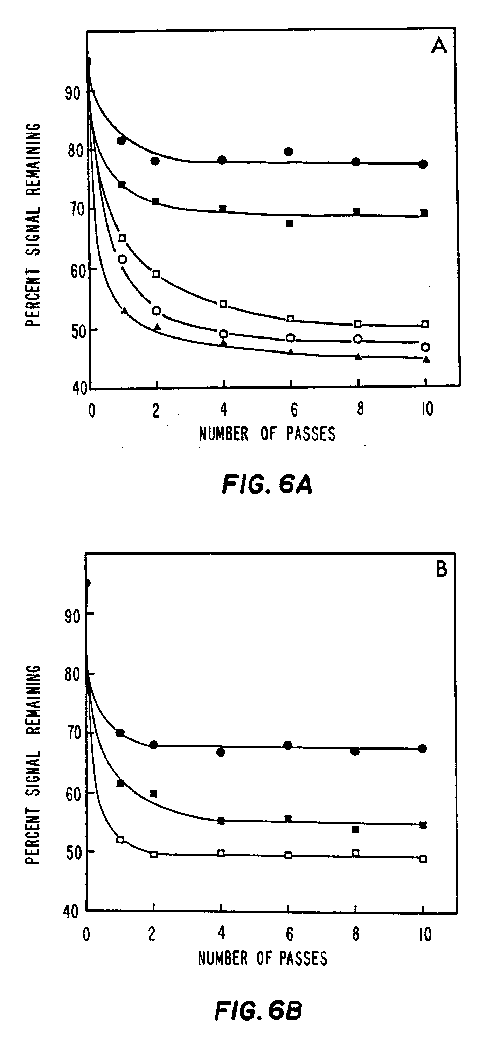

The influence of external Mn²⁺ on the ³¹P NMR signal intensity of EPC MLV systems (100 mg/ml) extruded through polycarbonate filters with pore sizes in the range 30 nm to 400 nm is illustrated in FIG. 6A. The signal intensity of the VET₁₀₀ systems decreases to 50%, indicating unilamellar character, after 8 passes through two stacked filters. Only 4 passes were found to be required to obtain predominantly unilamellar character when the lipid concentration is about 50 mg/ml or less. The VET₅₀ and VET₃₀ systems obtained on extrusion through the 50 and 30 nm pore size filters exhibit residual ³¹P NMR intensities of 48% and 44% respectively (after 8 passes) consistent with a unilamellar population of smaller vesicles.

The MLV systems extruded through the 200 nm and 400 nm filters retain multilamellar character as the residual ³¹P NMR signal intensities are 70 and 78% respectively. If it is assumed that the multilamellar vesicle population contain only two bilayers which are tightly packed (e.g., separated by 10 nm or less), then this would indicate that approximately 20% of the VET₂₀₀ systems are unilamellar and approximately 80% bilamellar. If the average number of lamellae is more than two, the proportion of unilamellar vesicle will be higher. Similar calculations for the VET₄₀₀ systems are consistent with nearly all the vesicles exhibiting bilamellar character, or with a small proportion of unilamellar vesicles which will increase for higher proportions of multilamellar vesicles with three or more bilayers.

FATMLVs exhibit significantly larger interlamellar spacings and much larger trapped volumes and VET₄₀₀ and VET₂₀₀ systems prepared from FATMLV's should exhibit somewhat higher unilamellar character due to the reduced fraction of tightly packed lamellae in the FATMLV precursors. This appears to be the case as illustrated in FIG. 6B, where it is shown that the residual ³¹P NMR intensities (after addition of Mn²⁺) for the VET₄₀₀ and VET₂₀₀ systems are 68% and 56% respectively. This would correspond to an average of approximately 20% unilamellar and 80% containing two (tightly packed) lamellae for the VET₄₀₀ systems and substantial population of unilamellar vesicles (at least 75%) for the VET₂₀₀ systems when prepared from FATMLV's. It may also be noted that fewer passes are required to generate the unilamellar LUVET₁₀₀ system from FATMLV's than from non-freeze thawed MLV's. Similar effects were observed for the SUVET₅₀ and SUVET₃₀ preparations.

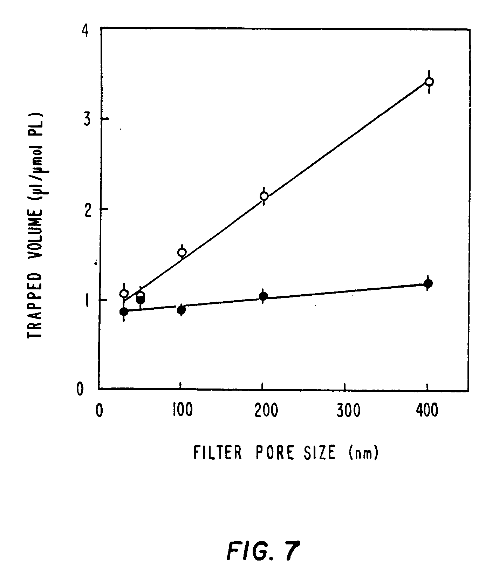

Assuming a similar size distribution, the increased unilamellar character of the VET₄₀₀ and VET₂₀₀ systems prepared from FATMLV's suggests that the trapped volume (expressed as liters trapped/ mol phospholipid) of freeze-thawed, sized vesicles should be significantly increased. That this is the case is illustrated in FIG. 7, where the trapped volume of the vesicles prepared from FATMLV's increases from 1 l/ mol to 3.6 l/ mol phospholipid as the pore size is increased from 30 to 400 nm. This contrasts strongly with the trapped volumes of the VET's prepared from MLV precursors, which increase by 20% or less for the same range of pore sizes. It is interesting to note that whereas the trapped volumes of the SUVET₃₀ and SUVET₅₀ vesicles are the same for FATMLV and MLV precursors, the trapped volumes of the LUVET₁₀₀ systems increase by 50% (to 1.5 l/ mol) when FATMLV's are employed. Comparable results were observed when ¹⁴C-inulin was used as the aqueous trap marker rather than ²²Na. Given that the ³¹P NMR data indicate unilamellar character for FATMLV and MLV systems extruded through filters with 100 nm pore size or less, it would not be expected that the trapped volumes observed should be sensitive to the freeze-thaw procedure.

The size distributions calculated by the procedure of van Venetie et al (see Methods and Materials) are given in Table 3 and reveal relatively homogeneous vesicle populations whose mean sizes are either somewhat smaller than the filter pore size (e.g., the VET₄₀₀ and VET₂₀₀ systems) or of the same size or larger than the filter pore size (e.g., the SUVET₅₀ and SUVET₃₀ systems). The size of the VET₂₀₀ vesicles is likely related to the fact that the actual pore sizes of the Nucleopore membranes are approximately 10% smaller than specified, whereas the sizes of the SUVET₅₀ and SUVET₃₀ systems may reflect a limiting size of the VET systems.

The vesicle sizes determined by light scattering techniques are also given in Table 3.

The results indicate that homogeneously-sized vesicles of SUV, LUV or MLV character can be readily generated by extruding MLV's or FATMLV's through polycarbonate filters of appropriate pore size.

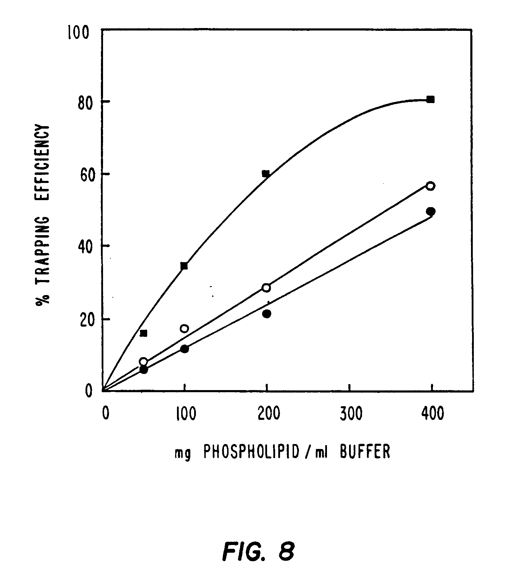

In FIG. 8, trapping efficiencies of 81%, 56% and 50% are obtained for FATMLV's (prepared at 400 mg/ml) extruded through 400 nm, 100 nm and 50 nm filters respectively. These trapping efficiencies are clearly remarkable, particularly for the smaller VET₁₀₀ and VET₅₀ systems. Freeze-fracture micrographs of VET₁₀₀ systems obtained at 400 mg/ml and then diluted to 100 mg/ml show that the size distribution is similar to that observed for the LUVET₁₀₀ systems prepared at 100 mg/ml and the low frequency of cross-fractures supports a unilamellar character.

When injected directly into the bloodstream, small vesicles are generally less leaky and remain in the blood for a longer time than larger systems.

Liposomes containing bioactive agents prepared in accordance with the present invention can be administered by the known routes of administration including orally, topically and parenterally. The dosage of the bioactive agent will generally be that for the free bioactive agent. The actual amount of bioactive agent administered will depond on a number of factors including the weight, age and condition of the patient. The physician will determine the actual dose administered.

Virtually any bioactive agent can be entrapped within the liposomes for use according to the present invention. Such agents include but are not limited to antibacterial compounds such as gentamycin, antiviral compounds such as rifampacin, antifungal compound such as amphotericin B, anti-parasitic compounds such as antimony derivatives, antineoplastic compounds such as vinblastine, vincristine, mitomycin C, doxorubicin, daunomycin, methotrexate, and cisplatinum, among others, proteins such as albumin, toxins such as diptheria toxin, enzymes such as catalase, hormones such as estrogens, neurotransmitters such as acetylcholine, lipoproteins such as alpha-lipoprotein, glycoproteins such as hyaluronic acid, immunoglobulins such as IgG, immunomodulators such as the interferons or the interleukens, dyes such as Arsenazo III, radiolabels such as ¹⁴C, radio-opaque compounds such as ⁹⁰Te, fluorescent compounds such as carboxy fluoroscein, polysaccharides such as glycogen, cell receptor binding molecules such as estrogen receptor protein, non-steroidal anti-inflammatories such as indomethacin, salicylic acid acetate, ibuprofen, sulindac, piroxicam, and naproxen; anti-inflammatories such as dexamethasone, antiglaucomic agents such as timolol or pilocarpine, anesthetics such as dibucaine, nucleic acids such as thymine, polynucleotides such as RNA polymers.

### MATERIALS AND METHODS

Egg phosphatidylcholine (EPC) was purified from hen egg yolks according to established procedures (see Singleton et. al., Journal of the American Oil Chemical Society, 42, 53 (1965) ) and was chromatographically pure. ²²NaCl and ¹⁴C-inulin were obtained from NEN Canada, Quebec.

Extrusion of the MLV or FATMLV preparations through two (stacked) polycarbonate filters of the various pore sizes (30-400 nm) was performed employing nitrogen pressures of up to 800 psi 5520 KPa. ³¹P NMR spectra of egg PC liposomes were obtained employing a Bruker WP-200 spectrometer operating at 81.0 MHz. Free induction decays corresponding to 1000 transients were accumulated utilizing 15 usec 90° radio frequency pulse, gated proton decoupling and a 20 KHz sweep width. An exponential multiplication corresponding to a 40 Hz line broadening was applied prior to Fourier transformation. Signal intensities were determined by cutting and weighing spectra.

The size distributions of the extruded liposomal systems were determined by freeze-fracture microscopy and quasi-elastic light scattering. Vesicle preparations to be used for freeze-fracture were mixed with glycerol (25% by volume) and frozen in a Freon slush. Samples were fractured and replicas obtained employing a Balzers BAF 400D apparatus, and micrographs of the replicas were produced using a Phillips' 400 electron microscope. Vesicle size distributions were estimated by measuring the diameter of fractured vesicles exhibiting 50% shadowing according to the procedure of van Venetie et al, Journal of Microscopy, 118, 401-408 (1980). Size distributions determined by quasi-elastic light scattering (QELS) analysis was performed utilizing a Nicomp Model 200 Laser Particle Sizer with a 5 milliwatt Helium-Neon Laser at an exciting wavelength of 632.8 nm. QELS, also referred to as dynamic light scattering or photon correlation spectroscopy, employs digital autocorrelation to analyze the fluctuations in scattered light intensity generated by the diffusion of vesicles in solution. The measured diffusion coefficient is used to obtain the average hydrodynamic radius and hence the mean diameter of the vesicles.

Vesicle trapped volumes were determined as follows: Phospholipid vesicles were hydrated and dispersed in the presence of tracer amounts of ²²NaCl or ¹⁴C-inulin (1 uCi/ml 3,7 x 10⁴Bg/ml). Subsequent to the extrusion process the vesicles were diluted to 100 mg/ml (when necessary) and passed down a Sephadex 650 or Ultrogel (LKB AcA-34) column to remove untrapped ²²Na+ or ¹⁴C-inulin, respectively. Aliquots of the vesicle-containing fraction were assayed for lipid phosphorus (15) and monitored for ²²Na+ utilizing a Beckman 8000 gamma counter or ¹⁴C-inulin using a Phillips' PW-4700 liquid scintillation counter. Trapped volumes were calculated and expressed as ul of aqueous trapped volume per umol of phospholipid. Trapping efficiencies were calculated as the dpm/umol phospholipid after gel filtration divided by the dpm/umol phospholipid before the gel filtration step.

### EXAMPLE 1

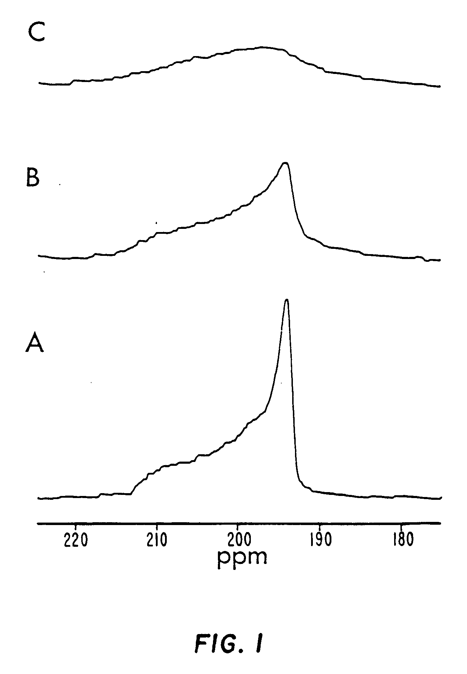

FIGS. 1 (A) and (B) show the 81.0 MHz ³¹P NMR spectra of egg phosphatidylcholine multilamellar vesicles of the prior art dispersed (A) in the absence of Mn²⁺ and (B) in the presence of 0.5 mM Mn²⁺. The spectra of (C) was obtained from the MLV's dispersed in the presence of 0.5 mM Mn²⁺ which were subsequently subjected to 5 freeze-thaw cycles employing liquid nitrogen and a 40°C water constant temperature bath in order to obtain vesicles of the present invention. The phosphatidylcholine was isolated from egg yolks employing standard procedures and was more than 99% pure as indicated by thin layer chromatography. The MLV's were prepared by adding 2 ml of buffer (150 mM sodium chloride, 20 mM Hepes, pH 7.5) to 200 mg of lipid. This dispersion was vortexed intermittently (2 min. vortexing, 3 min. interval) over 20 min. The ³¹P NMR spectra were collected at 20°C. employing a Bruker WP 200 spectrometer utilizing a 20 KHz sweep width, 2 sec interpulse delay and broad band proton decoupling.

### EXAMPLE 2

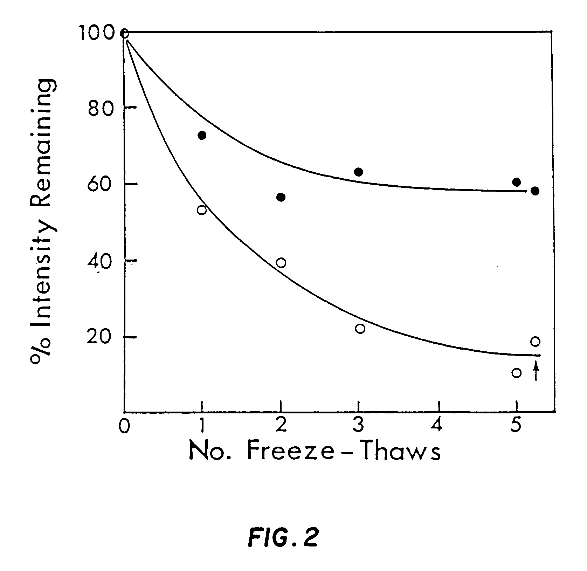

The influence of the number of liquid nitrogen 40°C water constant temperature bath freeze-thaw cycles on the ³¹P NMR signal intensity of egg phosphatidylcholine multilamellar vesicles prepared in the presence of 0.5 mM Mn²⁺ (closed circle) or 5 mM Mn²⁺ (open circle) are shown in FIG. 2. The samples were prepared using the materials and procedures of Example 1. The signal intensities were obtained by cutting and weighing the normalized spectra. The arrow indicates the signal intensity obtained after addition of sufficient aqueous Triton X-100 (10%, wt per vol) to solubilize the sample.

### EXAMPLE 3

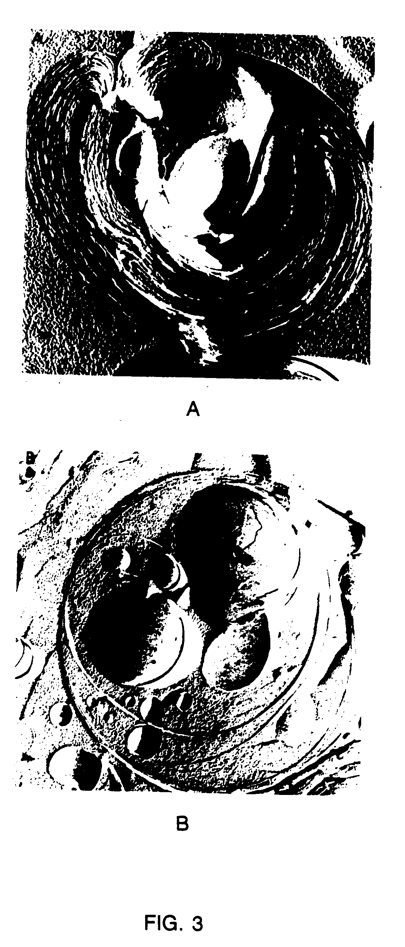

Freeze fracture electron micrographs of MLV's before (A) and after (B) five liquid nitrogen 40°C water constant temperature bath freeze-thaw cycles are shown in FIG. 3. The phospholipid concentration was 100 mg/ml. The samples were prepared using the procedures and materials of Example 1. The arrow indicates the direction of shadowing and the bar represents 140 nm.

### COMPARATIVE EXAMPLE 1

Two vesicle preparation procedures were employed. The first, as described in Example 1, entailed dispersion of dry egg phosphatidylcholine (PC) in 20 mM Hepes, 150 mM sodium chloride (pH 7.5) by vortex mixing followed by five freeze-thaw cycles employing liquid nitrogen and a 40°C water constant temperature bath.

The second procedure was that of Westman et al., Biochemica et Biophysica Acta, 685, 315-328 (1982), which utilized egg PC dried down to a thin film on the walls of a glass test tube as the starting material. To the thin film was added a sufficient amount of an unbuffered 0.1M sodium chloride solution containing 0, 2 or 4 mg tetracaine/ml to yield a final lipid concentration of 50 mg/ml. The lipid is then dispersed by vortex mixing. The pH of the sample is then adjusted to 7.5 using sodium hydroxide or hydrochloric acid followed by five freeze-thaw cycles employing ethanol/dry ice and a 40° water constant temperature bath.

Trapped volumes and trapping efficiencies were determined by including ²²Na⁺ (1 uCi/ml 3,7 x 10⁴Bg/ml) in the aqueous phase in which the lipid was dispersed. After the multilamellar vesicles were formed, aliquots were assayed for lipid phosphorus and ²²Na⁺ untrapped ²²Na⁺ was removed by washing with ²²Na⁺-free buffer employing low speed centrifugation. This procedure was repeated until supernatant counts were reduced to background levels. Aliquots of the pellet were then assayed for ²²Na⁺ and lipid phosphorous. Trapping efficiencies were calculated as the ratio of the cpm per umol lipid after and before removal of untrapped ²²Na⁺. Standard deviations, when given, were calculated from results obtained from 3 samples.

³¹Phosphorus-NMR spectra were collected at 20°C employing a Bruker WP200 spectrometer utilizing a 10 KHz sweep width, 2 sec interpulse delay and broad band proton decoupling. Peak intensities were obtained by cutting and weighing before and after addition of MnCl₂ in sufficient amounts to totally quench the externally exposed phospholipid signal.

Freeze-fracture replicas were prepared by mixing vesicle preparations (adjusted to 50 mg/ml) with glycerol (25% by volume), freezing samples in a freon slush and fracturing them employing a Balzers BAF 400D apparatus. Micrographs were obtained by using a Phillips 400 electron microscope.

Table 1 summarizes the trapped volume, trapping efficiency and ³¹P-NMR characteristics for MLV's and FATMLV's produced at varying concentrations by the procedures of the present invention and those produced by the Westman, et. al. procedure at 50 mg egg PC/ml in the presence of 0, 2 and 4 mg tetracaine/ml. Five freeze-thaw cycles employing liquid nitrogen dramatically increases the trapped volume and corresponding trapping efficiency of FATMLV preparations as compared to the non-freeze-thawed counterpart. This trapped volume increase is greater than 10-fold for FATMLVs prepared at 50 and 100 mg egg PC/ml while FATMLV's prepared at 200 and 400 mg egg PC/ml exhibit increases of 6.5- and 3.7-fold, respectively. The less dramatic trapped volume increases observed for FATMLV systems prepared at 200 and 400 mg egg PC/ml are likely due to the limited avaiIability of aqueous phase where 76.7 and 88.6% of the sample volume, respectively, is contained within the FATMLVs. The freeze-thaw protocol employed by Westman et al. also increases the trapped volume of FATMLVs but not to the extent observed for our systems (1.92, 2.23 and 3.44 ul/umol egg PC in the presence of 0, 2 and 4 mg tetracaine/ml, respectively, compared with 5.02 ul/umol egg PC with our preparation). Table 1 also demonstrates that the trapping efficiencies of MLV's and Westman FATMLV's are significantly lower than those observed for FATMLV's of the present invention.

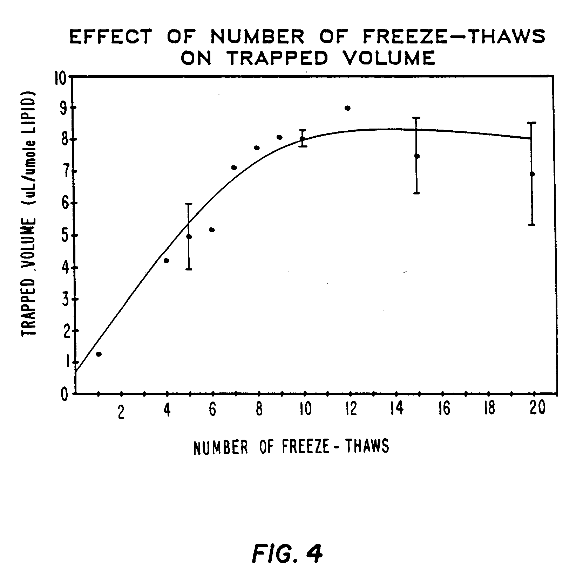

³¹P-NMR and freeze-fracture electron microscopy results corroborate the relationship of trapped volumes amoung the various vesicle preparations. The proportion of lipid in the outermost bilayer of vesicles can be determined by multiplying the percent ³¹P-NMR signal removed with Mn²⁺ addition by a factor of 2. As demonstrated in FIG. 4, FATMLV's produced by our procedure contain approximately 33% of the lipid in the outermost bilayer, a factor of 2 and 3 greater than Westman FATMLV's and standard MLVs, respectively. This result indicates that MLVs and Westman vesicles contain more lamellae per vesicle than do FATMLV's of the present invention produced at 50 mg/ml and is consistent with the lower trapped volumes observed for the prior systems.

In summary, the FATMLVs produced according to the Westman procedure and FATMLVs prepared by our protocol exhibit three major differences in physical characteristics. First, ours exhibit a 1.4- to 2.2-fold greater trapped volume than Westman's. Second, our FATMLVs display greater trapping efficiencies than do Westman's when prepared at 50 mg egg PC/ml and trapping efficiencies approaching 90% can be achieved by increasing the lipid concentration. Third, our FATMLVs contain approximately 2-fold more lipid in the outermost bilayer than do Westman's.

### COMPARATIVE EXAMPLE 2

Results were compared using liquid nitrogen and dry ice/ethanol (ETOH) in the freezing and thawing of multilamellar vesicles.

Egg PC MLV's were prepared at a concentration of 50 mg/ml in 20 mM Hepes, 150 mM NaCl (pH 7.5) by vortex mixing for approximately 5 min. in the presence of ³H-inulin as an aqueous marker. In the cases where lipid was used as a film, 50 mg egg PC was dissolved in 0.25 ml chloroform, the chloroform was removed with a stream of nitrogen and residual solvent was removed by high vacuum. The MLV's were subsequently frozen and thawed five times employing either a dry ice/ethanol bath or liquid nitrogen as indicated and a 40°C. water constant temperature bath. Aliquots were then taken and analyzed for lipid-phosphorus and radioactivity. Free (unentrapped) ³H-inulin was removed by washing the vesicles employing low speed centrifugation until supernatant counts were reduced to background levels. Aliquots were again taken and analyzed for lipid phosphorus and radioactivity. Trapping efficiencies were calculated as the dpm/umol egg PC after removal of free inulin divided by the dpm/umol egg PC obtained before the washing procedure. Results are shown in Table 2.

### EXAMPLE 4

FIG. 4 shows the effects of the number of freeze-thaw cycles on the trapped volume of FATMLV's of the present invention. MLV's were prepared from powdered egg PC in 20 mM Hepes, 150 mM sodium chloride (ph 7.5) as described in Example 1. The samples were frozen and thawed the indicated number of times employing liquid nitrogen and a 40°C water constant temperature bath. The samples (50 mg egg PC/ml) were assayed for lipid phosphous and radioactivity before and after removal of unentrapped ³H-inulin as described in Comparative Example 2. Vesicle trapped volume increased dramatically between zero and eight freeze thaw cycles from 0.5 to 8 ul/m mole egg PC, respectively. Increasing the number of freeze-thaw cycles from 8 to 20 resulted in no significant increase in FATMLV trapped volume.

### EXAMPLE 5

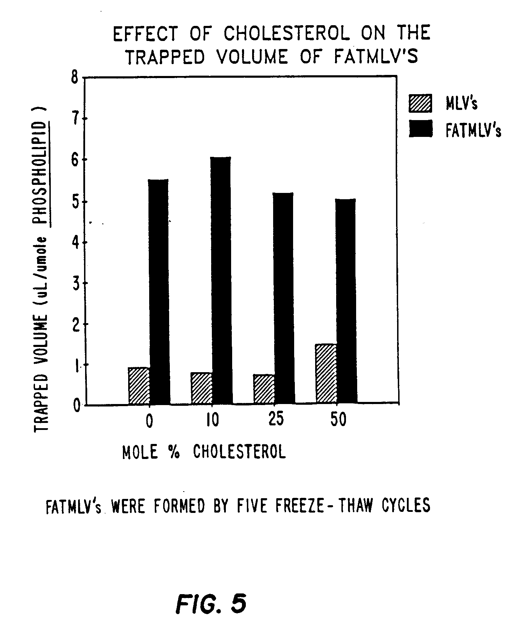

FIG. 5 shows the effect of cholesterol content on the trapped volume of FATMLV's of the present invention. Varying amounts of egg PC and cholesterol as indicated in FIG. 6 were dissolved in 0.5 ml chloroform and dried to a thin film on the walls of glass test tubes utilizing a stream of nitrogen and high vacuum. When no cholesterol was present, the concentration of egg PC was 50 mg/ml. MLV's were then prepared in the presences of 20 mM Hepes, 150 mM sodium chloride (pH 7.5) containing ³H-inulin as described in Example 1. FATMLV's were produced by freeze thawing the MLV's five times employing liquid nitrogen and a 40°C. constant temperature bath. Removal of unentrapped ³H-inulin and determination of vesicle trapped volumes were accomplished as described in Comparative Example 2.

### Example 6

FATMLV's using tobramycin phosphate (100 mg/ml), egg PC (300 mg/ml), at a pH of less than 3, preferably between 2 and 3, resulted in a 43.1% trapping efficiency. Trapping efficiencies can be increased by reducing the tobramycin phosphate concentration. Tobramycin does not act as an ideal aqueous marker because tobramycin interacts with the PC headgroup. The procedures of Example 1 were employed. An aqueous solution (1 ml) of tobramycin adjusted to a pH of 2.0 with phosphoric acid was added to 300 mg of egg PC and dispersed with vortexing. The samples were freeze-thawed five times; sized to 400 nm with five passes through two stacked 400 nm pore-sized polycarbonate filters at a pressure of 200 p.s.i. (1.380 KPa), freeze-thawed an additional two times and extruded an additional five times through two stacked polycarbonate filters having a 400 nm pore size. A sized tobramycin-liposome composition resulted.

### Example 7

FATMLV's were prepared from egg PC (400 mg/ml), pilocarpine hydrochloride (40 mg/ml, pH=4.1) according to the procedure of Example 1. The FATMLV had a trapping efficiency of 88.6 percent and a trapped volume of 1.77 ul/umol phospholipid. Homogeneously sized vesicles were prepared by extruding the FATMLV ten times through two stacked polycarbonate filters with a 50 nm pore size. Sized pilocarpine-containing vesicles resulted.

**Table 1**

| Physical Characteristics of MLV's, Westman FATMLV's and FATMLV's of the Present Invention | | | | |
|---|---|---|---|---|
| Sample | Lipid Concentration (mg/ml) | Trapped Volume (ul/umol lipid) | Trapping Efficiency (%) | %³¹P NMR Signal Removed with Mn²⁺ |
| MLV | 100 | 0.47 ± 0.03 | 5.8 | 5.0 |
| Westman FATMLV (0 mg tetracaine) | 50 | 1.92 ± 0.5 | 12.0 | 14.0 |
| Westman FATMLV (2 mg tetracaine) | 50 | 2.23 | 13.9 | 18.5 |
| Westman FATMLV (4 mg tetracaine) | 50 | 3.44 | 21.5 | 18.8 |
| FATMLV | 50 | 5.02 ± 0.04 | 31.3 | 16.6 |
| FATMLV | 100 | 5.27 ± 0.17 | 65.9 | 14.7 |
| FATMLV | 200 | 3.07 ± 0.05 | 76.7 | 7.4 |
| FATMLV | 400 | 1.77 ± 0.09 | 88.6 | 7.2 |

**Table 2**

| Sample | Freezing Media | Trapped Volume (ul/umol EPC) | Trapping Efficiency (%) |
|---|---|---|---|
| EPC (powder) | dry ice/ETOH | 3.44 | 21.5 |
| EPC (film) | dry ice/ETOH | 3.62 | 22.6 |
| EPC (powder) | liquid nitrogen | 6.81 | 42.5 |
| EPC (film) | liquid nitrogen | 5.54 | 34.6 |

**Table 3**

| Size Distributions of Extruded Vesicles^{a} | | |
|---|---|---|
| Filter pore size (nm) | Mean Diameter ± S.D. (nm) | |
| | Freeze-fracture electron microscopy^{b} | Quasielastic light scattering^{c} |
| 400 | 243 ± 91 | N.D.^{d} |
| 200 | 151 ± 36 | 179.9 ± 55 |
| 100 | 103 ± 20 | 138.7 ± 36 |
| 50 | 68 ± 19 | 73.8 ± 18 |
| 30 | 56 ± 17 | 63.1 ± 17 |

| | | |
|---|---|---|
| ^{a} MLVs (100 mg EPC/ml) were frozen and thawed 5 times prior to the extrusion process. These FATMLVs were then passed 20 times through 2 (stacked) polycarbonate filters of the indicated pore size. | | |
| ^{b} Size distribution analysis of vesicle employing freeze-fracture electron microscopy were completed as described in Materials and Methods. Mean diameters and S.D. were determined by measuring 150 vesicles. | | |
| ^{c} Size distribution analysis of vesicles employing quasielastic light scattering were completed as described in Materials and Methods. | | |
| ^{d} Statistical analysis of the data yielding low fit error could not be accomplished. A mean vesicle diameter value is therefore not reported. | | |

## Claims

1. A process for preparing a multilamellar vesicle, containing a bioactive substance, having interlamellar equal solute distribution, dispersed in an aqueous phase in the absence of organic solvent or detergents comprising the steps of : (a) dispersing a lipid in an aqueous solvent to form a multilamellar vesicle ; (b) rapidly freezing the multilamellar vesicle at about -196°C to obtain a frozen lipid-aqueous medium mixture ; (c) warming the mixture in a constant temperature bath to melt the aqueous medium ; and (d) repeating steps (b) and (c) at least two times.

2. The process of claim 1 wherein liquid nitrogen is employed in step (b).

3. The process of claims 1 to 2 wherein steps (b) and (c) are performed at least five times.

4. The process of claims 1 to 3 comprising the additional step of concentrating the multilamellar vesicle dispersion by methods comprising dialysis, centrifugation and dehydration.

5. The process of claims 1 to 4 comprising the additional step of filtering the multilamellar vesicle dispersion.

6. The process of claim 5 comprising the additional step of filtering the multilamellar vesicle dispersion through a polycarbonate filter.

7. A concentrated composition obtained by the method of claim 5.

8. A multilamellar vesicle obtainable by anyone of claims 1 to 6 having interlamellar equal solute distribution dispersed in an aqueous phase comprising an aqueous medium, a lipid concentration of at least 50 mg/ml and a trapping efficiency of at least 30 percent.

9. The vesicle of claim 8 wherein the trapping efficiency is at least about 40 percent.

10. The vesicle of claim 8 or 9 wherein the lipid concentration is at least about 100 mg/ml and the trapping efficiency is at least about 50 percent.

11. The vesicle of claims 8 to 10 wherein the lipid concentration is between about 100 and 1000 mg/ml.

12. The vesicle of claims 8 to 11 having equal solute distribution within the aqueous medium.

13. The vesicle of claims 8 to 12 wherein the lipid comprises phospholipid.

14. The vesicle of claim 13 wherein the phospholipid comprises phosphatidylcholine.

15. The vesicles of claims 13 and 14 where in the lipid additionally comprises a sterol.

16. The vesicle of claim 15 wherein the sterol is cholesterol.

17. Multilamellar vesicles containing a bioactive agent, having interlamellar equal solute distribution according to any one of claims 8 to 16, obtainable by the process of claims 3 to 5 in which steps (b) and (c) are performed at least five times characterized in that they have closed lamellar systems intercalated between bilayers.

18. The multilamellar vesicle of any one of claims 8 to 17 wherein the bioactive agent is hydrophilic.

19. The multilamellar vesicle of claims 8 to 17 wherein the bioactive agent is lipophilic.

20. The multilamellar vesicle of claim 19 wherein the bioactive agent is tobramycin or pilocarpine.

21. The multilamellar vesicle of claim 19 wherein the bioactive agent is a phosphate salt of tobramycin or hydrochloride pilocarpine.

## Patentansprüche

1. Verfahren zur Herstellung eines multilamellaren Vesikels, das eine bioaktive Substanz enthält, mit einer interlamellaren gleichen Verteilung des Gelösten, dispergiert in einer wässrigen Phase in Abwesenheit eines organischen Lösungsmittels oder von Detergentien, das die Schritte umfaßt: (a) Dispergieren eines Lipids in einem wässrigen Lösungsmittel, um ein multilamellares Vesikel zu bilden; (b) schnelles Einfrieren des multilamellaren Vesikels auf ca. -196°C, um eine gefrorene Lipid-wässriges Medium-Mischung zu erhalten; (c) Erwärmen der Mischung in einem Bad mit konstanter Temperatur, um das wässrige Medium zu schmelzen; und (d) wenigstens zweimaliges Wiederholen der Schritte (b) und (c).

2. Verfahren nach Anspruch 1, worin in Schritt (b) flüssiger Stickstoff verwendet wird.

3. Verfahren nach den Ansprüchen 1 bis 2, worin die Schritte (b) und (c) mindestens fünfmal ausgeführt werden.

4. Verfahren nach den Ansprüchen 1 bis 3, das den zusätzlichen Schritt der Konzentrierung der multilamellaren Vesikeldispersion umfaßt, durch Verfahren, die die Dialyse, Zentrifugation und Dehydratation umfassen.

5. Verfahren nach den Ansprüchen 1 bis 4, den als zusätzlichen Schritt des Filtrierens der multilamellaren Vesikeldispersion umfaßt.

6. Verfahren nach Anspruch 5, das den zusätzlichen Schritt des Filtrierens der multilamellaren Vesikeldispersion durch einen Polycarbonatfilter umfaßt.

7. Konzentrierte Zusammensetzung, erhalten durch das Verfahren nach Anspruch 5.

8. Multilamellares Vesikel, erhältlich durch jeden der Ansprüche 1 bis 6, mit einer interlamellaren gleichen Verteilung des Gelösten, dispergiert in einer wässrigen Phase, das ein wässriges Medium, eine Lipidkonzentration von mindestens 50 mg/ml und einen Einfangkoeffizienten von mindestens 30% aufweist.

9. Vesikel nach Anspruch 8, worin der Einfangkoeffizient mindestens ca. 40% beträgt.

10. Vesikel nach Anspruch 8 oder 9, worin die Lipidkonzentration mindestens 100 mg/ml beträgt und der Einfangkoeffizient mindestens 50% beträgt.

11. Vesikel nach den Ansprüchen 8 bis 10, worin die Lipidkonzentration zwischen ca. 100 und 1000 mg/ml liegt.

12. Vesikel nach den Ansprüchen 8 bis 11 mit gleicher Verteilung des Gelösten innerhalb des wässrigen Mediums.

13. Vesikel nach den Ansprüchen 8 bis 12, worin das Lipid ein Phospholipid umfaßt.

14. Vesikel nach Anspruch 13, worin das Phospholipid Phosphatidylcholin umfaßt.

15. Vesikel nach den Ansprüchen 13 und 14, worin das Lipid zusätzlich ein Sterol umfaßt.

16. Vesikel nach Anspruch 15, worin das Sterol Cholesterol ist.

17. Multilamellare, ein bioaktives Mittel enthaltende Vesikel mit einer interlamellaren gleichen Verteilung des Gelösten nach einem der Ansprüche 8 bis 16, erhältlich durch das Verfahren nach den Ansprüchen 3 bis 5, worin die Schritte (b) und (c) mindestens fünfmal ausgeführt werden, dadurch charakterisiert, daß sie geschlossene lamellare Systeme, eingelagert zwischen molekularen Doppelschichten aufweisen.

18. Multilamellares Vesikel nach jedem der Ansprüche 8 bis 17, worin das bioaktive Mittel hydrophil ist.

19. Multilamellares Vesikel der Ansprüche 8 bis 17, worin das bioaktive Mittel lipophil ist.

20. Multilamellares Vesikel nach Anspruch 19, worin das bioaktive Mittel Tobramycin oder Pilocarpin ist.

21. Multilamellares Vesikel nach Anspruch 19, worin das bioaktive Mittel ein Phosphatsalz von Tobramycin oder Pilocarpin-Hydrochlorid ist.

## Revendications

1. Procédé pour la préparation d'une vésicule multilamellaire, contenant une substance bioactive, ayant une distribution interlamellaire égale du soluté, dispersée dans une phase aqueuse en l'absence de solvant organique ou de détergents, comprenant les étapes de: (a) dispersion d'un lipide dans un solvant aqueux pour former une vésicule multilamellaire; (b) congélation rapide de la visécule multilamellaire à environ -196°C pour obtenir un mélange lipide-milieu aqueux congelé; (c) chauffage du mélange dans un bain à température constante pour faire fondre le milieu aqueux; et (d) répétition des étapes (b) et (c) au moins deux fois.

2. Procédé selon la revendication 1, dans lequel de l'azote liquide est employé dans l'étape (b).

3. Procédé selon l'une des revendications 1 et 2, dans lequel les étapes (b) et (c) sont mises en oeuvre au moins cinq fois.

4. Procédé selon l'une quelconque des revendications 1 à 3, comprenant l'étape additionnelle de concentration de la dispersion de vésicules multilamellaires par des méthodes comprenant la dialyse, la centrifugation et la déshydratation.

5. Procédé selon l'une quelconque des revendications 1 à 4, comprenant l'étape additionnelle de filtration de la dispersion de vésicules multilamellaires.

6. Procédé selon la revendication 5, comprenant l'étape additionnelle de filtration de la dispersion de vésicules multilamellaires à travers un filtre en polycarbonate.

7. Composition concentrée obtenue par le procédé selon la revendication 5.

8. Vésicule multilamellaire pouvant être obtenue selon l'une quelconque des revendications 1 à 6, ayant une distribution interlamellaire égale du soluté, dispersée dans une phase aqueuse comprenant un milieu aqueux, une concentration en lipide d'au moins 50 mg/ml et une efficacité de capture d'au moins 30 pour cent.

9. Vésicule selon la revendication 8, dans laquelle l'efficacité de capture est d'au moins 40 pour cent.

10. Vésicule selon l'une des revendications 8 ou 9, dans laquelle la concentration en lipide est d'au moins environ 100 mg/ml et l'efficacité de capture est d'au moins environ 50 pour cent.

11. Vésicule selon l'une quelconque des revendications 8 à 10, dans laquelle la concentration en lipide est comprise entre environ 100 et 1000 mg/ml.

12. Vésicule selon l'une quelconque des revendications 8 à 11, ayant une distribution égale du soluté dans le milieu aqueux.

13. Vésicule selon l'une quelconque des revendications 8 à 12, dans laquelle le lipide comprend un phospholipide.

14. Vésicule selon la revendication 13, dans laquelle le phospholipide comprend de la phosphatidylcholine.

15. Vésicules selon l'une des revendications 13 et 14, dans lesquelles le lipide comprend en outre un stérol.

16. Vésicule selon la revendication 15, dans laquelle le stérol est du cholestérol.

17. Vésicules multilamellaires contenant un agent bioactif, ayant une distribution interlamellaire égale du soluté selon l'une quelconque des revendications 8 à 16, pouvant être obtenues par le procédé selon l'une quelconque des revendications 3 à 5, dans lequel les étapes (b) et (c) sont mises en oeuvre au moins cinq fois, caractérisées en ce qu'elles possèdent des systèmes lamellaires fermés intercalés entre des couches doubles.

18. Vésicule multilamellaire selon l'une quelconque des revendications 8 à 17, dans laquelle l'agent bioactif est hydrophile.

19. Vésicule multilamellaire selon l'une quelconque des revendications 8 à 17, dans laquelle l'agent bioactif est lipophile.

20. Vésicule multilamellaire selon la revendication 19, dans laquelle l'agent bioactif est la tobramycine ou la pilocarpine.

21. Vésicule multilamellaire selon la revendication 19, dans laquelle l'agent bioactif est un phosphate de tobramycine ou un chlorhydrate de pilocarpine.
